# EUROPEAN PATENT APPLICATION

(11) **EP 0 711 532 A1**
(43) Date of publication of application: **15.05.1996**
(21) Application number: 95308099.1
(22) Date of filing: 13.11.1995
(51) Int. Cl.: A61B 17/12

(54) **Delivery device**

(30) Priority: 14.11.1994 US 338377
(71) Applicant: TARGET THERAPEUTICS, INC., Fremont, CA 94537-5120 (US)
(72) Inventor: Samson, Gene, Milpitas, California 95035 (US)
(74) Representative: Price, Nigel John King

(57) **Abstract**

This invention is a surgical device and a method of using it. It is a combination in which at least one vasoocclusive device, such as a coil, is placed directly on a core wire. The vasoocclusive coil (or coils) may be press-fit onto the core wire but are slidably removable using a pusher on the proximal end of the core wire. The core wire may also function as a guidewire by inclusion of a radiopaque, shapeable tip. This assembly may be used without an infusion or micro-catheter in the cerebral vasculature, although a guiding catheter is often used to direct the assembly to the region of the neck after introduction from an external access point.

## Description

### FIELD OF THE INVENTION

This invention is a coil delivery assembly. This assembly is a combination in which at least one vasoocclusive device, such as a coil, is placed directly on a core wire. The vasoocclusive coil (or coils) may be press-fit onto the core wire but are slidably removable using a pusher on the proximal end of the core wire. The core wire may also function as a guidewire by inclusion of a radiopaque, shapeable tip. This assembly may be used without an infusion or micro-catheter in the cerebral vasculature, although a guiding catheter is often used to direct the assembly to the region of the neck after introduction from an external access point.

### BACKGROUND OF THE INVENTION

Vasoocclusion devices, such as coils and braids, are used to occlude a variety of sites within the vasculature. For instance, such vasoocclusive devices are used to fill vascular cavities, such as aneurysms, or block blood supply to other sites such as vascular accidents (e.g., strokes) and arterio-venous malformations (AVMs). In each case, the vasoocclusive device forms an embolic occlusion which may, in turn, form a collagenous mass.

Currently available coils are normally delivered to the selected site through a series of catheters. For instance, when the selected site is in the brain, a typical procedure will first involve a large diameter (but short) introducer catheter introduced into the femoral artery in the groin area. A longer catheter -- a guiding catheter having a certain amount of stiffness -- is then placed through the introducer catheter and advanced towards the heart. Using the stiffness of the guiding catheter itself, and perhaps a guidewire, the guiding catheter is steered around the heart and into an appropriate artery leaving the aortic arch. The guiding catheter seldom is suitable for an approach to the brain. The soft tissue of the brain is liable to trauma if a catheter of high stiffness is pressed into the brain.

A micro-catheter (or infusion catheter) is then introduced into the guiding catheter to complete the complicated vascular journey into the brain.

Typical of the better designs for micro-catheters used for accessing the brain may be found in U.S. Patent No. 4,739,768, to Engelson, and U.S. Patent No. 4,813,934, to Sepetka et al. Each of these catheter designs have multiple sections with differing flexibilities. The most distal of the catheter sections in these designs is exceptionally flexible and able to follow a guidewire into extraordinarily complicated vasculature. The more proximal sections of the catheter are respectively more stiff.

Once the distal end of the micro-catheter is properly positioned at the selected vascular site, the guidewire is typically withdrawn. The vasoocclusive devices are then inserted into the micro-catheter lumen and pushed to (and out of) the distal end of the catheter to occlude that site.

There are a number of vasoocclusive devices and pushers which may be found in the patent literature.

Ritchart et al. (U.S. Patent No. 4,994,069) describes a basic method of placing and discharging a variety of vasoocclusive coils using a micro-catheter to place the coils in the vasculature.

Another such device includes the electrolytic detachment of a vasoocclusive coil from a pusher wire -- See, U.S. Patent Nos. 5,122,136 to Guglielmi et al. These devices use a micro-catheter to approach the selected vascular site.

Mechanically detachable vasoocclusive devices are discussed in U.S. Patent No. 5,234,437, to Sepetka. Sepetka describes a pusher which engages the vasoocclusive coil by threading into the proximal end of the coil. A sleeve is maintained over the threaded end of the pusher as it is unthreaded from the coil. The coil and pusher are directed to the selected vascular site by use of a micro-catheter.

Other mechanically detachable vasoocclusive coil assemblies which use micro-catheters to reach the selected vascular site include:
U.S. Pat. No. 5,250,071, to Palermo
U.S. Pat. No. 5,261,916, to Engelson
U.S. Pat. No. 5,304,195, to Twyford et al
U.S. Pat. No. 5,312,415, to Palermo
U.S. Pat. No. 5,350,397, to Palermo et al

None of these documents suggest the use of a guidewire with one or more tight-fitting vasoocclusive coils on that guidewire. Specifically, none of the documents teach a vasoocclusive coil delivery combination not having a micro-catheter.

### SUMMARY OF THE INVENTION

This invention is a vasoocclusive device, typically a coil, in combination with a core wire or guidewire. The coil(s) fits tightly onto the guidewire but may be removed by pushing it from its proximal end. The combination has sufficient flexibility and directability that it is used without a micro-catheter. It may be used in conjunction with a guide catheter, however.

The combination may be configured so that a sequence of vasoocclusive devices are placed on the guidewire and may be displaced at one or more chosen sites within the body, as needed or if needed.

This device is useful for introducing coils of any diameter but, as a practical matter, the upper diameter of such coils is 0.040" for most neurological applications. The inventive combination is especially suitable for introducing coils which have those larger diameters because the lack of a need for the micro-catheter.

The procedure for use involves introduction of a guide catheter into the body; introduction of a guide wire of the type described above (having one or more vasoocclusive coils helically wound about the guidewire) into the lumen of the guide catheter; extension of the guidewire beyond the guide catheter; push of one or more coils distally beyond the end of the guidewire; and withdrawal of the guidewire and guide catheter.

### BRIEF DESCRIPTION OF THE DRAWINGS

Figure 1 shows a magnified, side-view, cross-section of the inventive combination guidewire vasoocclusive device.

Figure 2 shows a magnified, side-view, cross-section of the inventive combination guidewire vasoocclusive device in conjunction with the pusher and the guide catheter.

Figures 3 and 4 show a comparison of the inventive device with the typical coil delivery catheter assembly.

### DESCRIPTION OF THE INVENTION

This invention is a combination suitable for delivering one or more vasoocclusive devices to a selected site in the vasculature. The invention also includes a method of using the combination.

In particular, the combination comprises a central guidewire (or core wire), typically with a formable tip and one or more embolic coils fitting tightly on the guidewire but still able to slide off the distal end of the guidewire upon urging from a pusher.

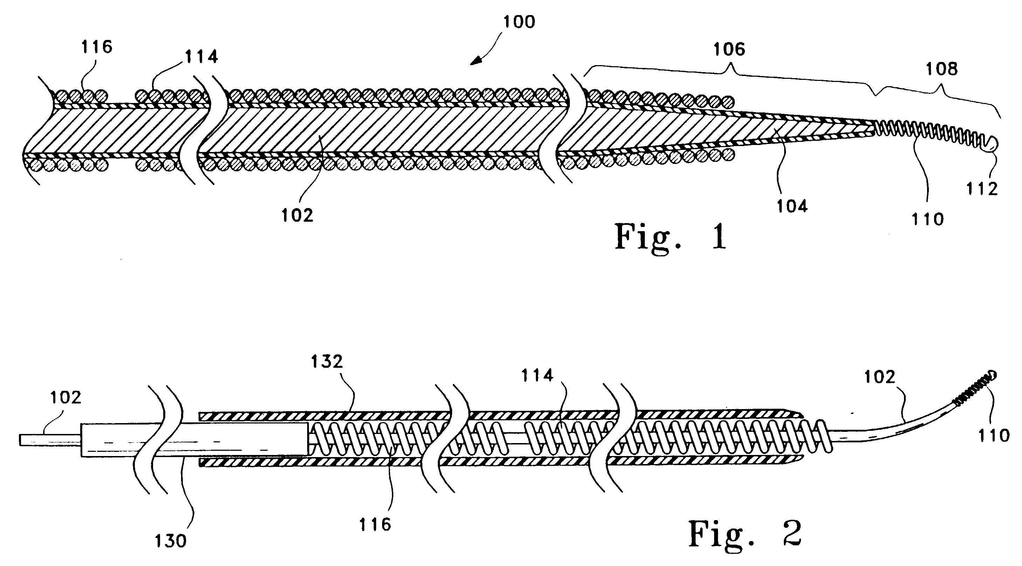

Figure 1 shows a cross-sectional view of the combination guidewire-vasoocclusive coil (100). The core wire (102) has both a distal and a proximal end. Also shown in Figure 1 is a coating (104) which may be a lubricious material such as a polyfluorocarbon (e.g., a Teflon). The core wire (102) may be of any suitable material but typically is metallic. Acceptable metals include many stainless steels (SS304, SS308, SS312, etc.) and superelastic alloys. An especially suitable superelastic alloy is a nickel-titanium alloy discovered by the U.S. Naval Ordinance Laboratory called nitinol. These materials are discussed at length in U.S. Patent Nos. 3,174,851 to Buehler et al., 3,351,463 to Rozner et al., and 3,753,700 to Harrison et al.

The use of a superelastic alloy permits a guidewire of smaller diameter and hence an inventive device more able to access very narrow vasculature.

Figure 1 also depicts a portion (106) of the core wire (102) having a taper. The core wire (102) may be tapered (or not, as desired) anywhere along its length.

The shapeable tip (108) in the depicted instance comprises a coil (110) which may be radiopaque. Suitable metals for the shapeable coil (108) which allow both shapeability and radiopacity include platinum, palladium, rhodium, gold, and other platinum-group metals. Alloys of these metals are also suitable. Platinum-tungsten alloys are especially useful.

The coil (110) often has a smooth end (112). The coil (110) may (or may not) include an interior section extending from the body of the core wire (102). Extension ribbons are a known variation for extending the body of the core wire to the tip of the shapeable tip (108).

The diameter of the core wire (112) may be anywhere between 0.005" to a practical diameter of about 0.040". The diameter is not particularly critical except to the extent it affects accessibility in the vasculature. The noted taper in the distal section (106) is for the purpose of increasing flexibility of the core wire 9102) in that section to allow enhanced accessibility.

Figure 1 shows an embolic coil (114) which is helically wound about the core wire (102). In the depicted variation, vasoocclusive coil (114) extends nearly to the distal end of the core wire (102). Also shown in Figure 1 is a second vasoocclusive coil (116). A single coil (114) may be pushed from the distal end of the core wire (102). Or, if so desired, a subsequent coil (116) may also be used from the core wire (102). Additional vasoocclusive coils (not shown) may be added to the combination if so desired.

The wire making up the various vasoocclusive coils may be from 0.0005" to 0.010" in diameter depending upon the use to which the coil is put. For instance, for occlusion of typical neural or peripheral sites, a diameter of 0.0005" to 0.003" might be appropriate. For occlusion of a Vein of Galen or an AVM, a stiffer coil of a larger diameter wire, e.g., 0.002" to 0.006", may be appropriate.

Figure 2 shows a simplified, sectional, partial cross-section of the inventive combination. The combination depicts, in addition to the components shown in Figure 1, a pusher (130) in proximal relationship to the multiple coils (114 & 116) and a guide catheter (132). Various necessary and well known portions of a typical catheter combination, e.g., luers and means for producing radiopacity (bands and the like), have been omitted for simplicity of portrayal. The core wire (102) is shown extending from both the proximal and distal ends of the inventive combination. A shapeable coil (110) is also shown. The pusher (130) is slidable over the core wire (102) and is in contact with the proximal end of the helically wound vasoocclusive coil (116). During deployment of the coils (114 & 116) into the vasculature, the core wire (102) is held in place and the pusher (130) is moved distally and axially along the core wire (102) allowing the coils (114 & 116) to slide off over the shapeable tip (110) and into the selected vascular site.

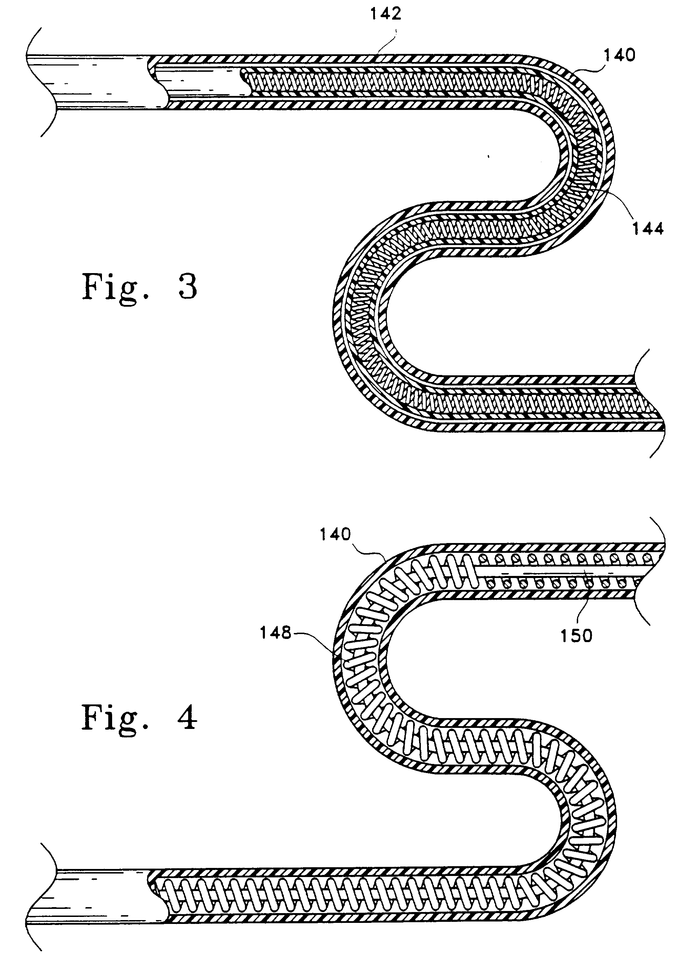

Finally, Figures 3 and 4 depict a major benefit in utilization of the invention described herein. Figure 3 shows the cross-section of a mid-section in a typical catheter assembly used in delivering a vasooclusive coil. Figure 4 shows that same cross-section as used in the inventive combination for delivering a vasoocclusive coil.

Figure 3 shows a guide catheter (140) which is similar in construction to the guide catheter (132) shown in Figure 2. Interior to the guiding catheter (140) is a micro-catheter (142) and a vasoocclusive coil (144). The diameter of the coil (144) is limited because of the presence of the micro-catheter (142) and the propensity for binding of the various components -- micro-catheter to guide catheter and coil to micro-catheter -- is enhanced just because of their flexibility and size. There is no guidewire inside the coil (144).

In contrast, Figure 4 shows a guide catheter (140) and, interior to the guiding catheter (140), a vasoocclusive coil (148) and a core wire (150). The absence of the micro-catheter in the inventive variation shown this Figure 4 allows more room for larger coils and lowers the propensity for binding in tight turns.

This invention has been described and specific examples of the invention have portrayed. The use of those specifics is not intended to limit the invention in any way. Additionally, to the extent that there are variations of the invention which are within the spirit of the disclosure and yet are equivalent to the inventions found in the claims, it is our intent that this patent cover those variations as well.

## Claims

1. A vasoocclusive coil delivery assembly comprising:
a) an elongated core wire having a proximal end and a distal end and a shapeable guide tip forming said distal end, and
b) at least one helically wound vasoocclusive coil fitting coaxially about said core wire and slidably distally removable from said core wire.

2. The assembly of claim 1, wherein at least a portion of the elongated core wire is tapered.

3. The assembly of claim 1 or claim 2, additionally comprising a pusher adapted to push said at least one helically wound vasoocclusive coil distally and off said core wire.

4. The assembly of claim 3, wherein the pusher comprises a tubular number located coaxially about said core wire and proximally of the at least one vasoocclusive coil and which is slidable on said core wire.

5. The assembly of any one of the preceding claims, further comprising a lubricious coating on at least a portion of said core wire.

6. The assembly of any one of the preceding claims, wherein the shapeable tip comprises a helically wound coil fixedly attached to said core wire.

7. The assembly of claim 6, wherein the helically wound shapeable tip is radiopaque.

8. The assembly of any one of the preceding claims, comprising more than one helically wound vasoocclusive coil.

9. The assembly of claim 8, wherein the most distal vasoocclusive coil has a length measured along the axis of the core wire which is greater than the remaining more proximal vasoocclusive coils.

10. A vasoocclusive coil delivery assembly comprising:
a) a metallic elongated core wire having a proximal end and a distal end,
b) a shapeable, radiopaque, helically wound coil guide top fixedly attached to said core wire distal end,
c) at least one helically wound vasoocclusive coil fitting coaxially about said core wire and slidably, distally removable from said guidewire, and
d) a tubular pusher member located coaxially about said core wire and proximally of said at least one helically wound vasoocclusive coils and adapted to push said vasoocclusive coils distally on said core wire.

11. The assembly of any one of the preceding claims, wherein the core wire comprises stainless steel.

12. The assembly of any one of the preceding claims, wherein the core wire comprises a superelastic alloy.

13. The assembly of claim 11, wherein the superelastic alloy comprises nitinol.

14. The assembly of any one of the preceding claims, additionally comprising a guiding catheter.

15. The assembly of claim 13, wherein the guiding catheter is located coaxially about at least a portion of said core wire and vasoocclusive coil.
